# EUROPEAN PATENT SPECIFICATION

(11) **EP 1 263 686 B1**
(45) Date of publication and mention of the grant of the patent: **29.11.2006**
(21) Application number: 00901267.5
(22) Date of filing: 01.02.2000
(51) Int. Cl.: C02F 3/28, C12N 13/00, B01D 53/14, B01D 53/18

(54) **PROCESS FOR PRODUCTION OF HYDROGEN FROM ANAEROBICALLY DECOMPOSED ORGANIC MATERIAL**
VERFAHREN ZUR HERSTELLUNG VON WASSERSTOFF AUS DER ANAEROBEN ZERSETZUNG VON ORGANISCHEN STOFFEN
PRODUCTION D'HYDROGENE A PARTIR DE MATIERES ORGANIQUES DECOMPOSEES PAR UN PROCEDE ANAEROBIE

(43) Date of publication of application: 11.12.2002
(73) Proprietor: Roychowdhury, Sukomal, San Diego, CA 92122 (US); McAlister, Roy E., Phoenix, AZ 85029 (US)
(72) Inventor: ROYCHOWDHURY, Sukomal, San Diego, CA92122 (US); McALISTER, Roy, Edward, Phoenix, AZ 85029 (US)
(74) Representative: HOFFMANN EITLE
(86) International application number: PCT/GB2000/000288
(87) International publication number: WO 2001/056938

(56) References cited:
- WO-A-94/08907
- GB-A- 2 076 849
- GB-A- 2 149 423
- GB-A- 2 190 682
- US-A- 4 053 395
- PATENT ABSTRACTS OF JAPAN vol. 1995, no. 05, 30 June 1995 (1995-06-30) & JP 07 031998 A (EBARA RES CO LTD;OTHERS: 01), 3 February 1995 (1995-02-03)

## Description

This invention relates to a process for the production of hydrogen from anaerobically decomposed organic materials, including anaerobically decomposed organic materials such as are found in landfill materials and sewage sludge.

It is recognized that additional sources of energy are needed for sustained industrial growth. There exists an ever present danger in depending too heavily on fossil fuels. Fossil fuels (hydrocarbons) represent a limited supply of stored energy which is typically released during a combustion process. By burning hydrocarbons mankind has spewed billions of tons of toxic pollutants into the atmosphere. It therefore makes sense from both environmental and economic standpoints to develop alternative sources of renewable fuels.

Hydrogen is a fuel which does not produce pollutants, water being its only combustion product. Hydrogen has many industrial uses, for example in the production of fertilizers, dyes, drugs, plastics, hydrogenated oils and fats and methanol, and is used in many industries. It is also used as a rocket fuel and may be used in accordance with the present invention as a minus-emissions fuel that allows ordinary engines to clean the air.

The established processes for producing commercially significant amounts of hydrogen are: (1) steam reforming of hydrocarbons, (2) partial oxidation of coal, (3) electrolysis of water, and (4) direct use of solar radiation (photovoltaic method).

Steam reformation of hydrocarbons and partial oxidation of coal are disadvantageous in that fossil hydrocarbon fuels are consumed. Production of hydrogen by electrolysis of water, a relatively simple and non-polluting process, is costly and therefore economically disadvantageous for most industrial applications, because the amount of energy needed for electrolysis of water exceeds the energy obtained from the combustion of the resulting hydrogen. Photovoltaic methods of hydrogen production are restricted by the limited access to solar radiation experienced by much of the world's population.

A method of producing hydrogen from sugars such as glucose or maltose is discussed in Energy and the Environment, Proceedings of the 1st World Renewable Energy Congress, Reading, UK 23-28 September 1990: S. Roychowdhury and D. Cox ("Roychowdhury"). This method involves preparation of "landfill inocula" from materials obtained from various depths in a landfill by drying, grinding (to obtain "landfill powder") and incubation *in situ.* The resulting incubated culture medium was observed primarily to produce carbon dioxide and methane, and little else, indicating the presence of highly methanogenic flora in the inocula. However, inoculation of various sugar solutions with supernatant from such culture media, or in certain cases with landfill powder, was found to lead to production of hydrogen and carbon dioxide, with no methane or oxygen being produced. This indicates the presence of hydrogen-producing bacteria in the landfill inocula and/or the influence of released hydrogen upon the landfill materials and inocula. Hydrogen production was observed to decrease with increasing acidity.

The present invention is based in part on the finding that anaerobically decomposed organic materials may be treated by passage therethrough of a relatively small and/or intermittent electric current in order to enhance production of hydrogen therefrom and to suppress formation of methane. Such treatment permits production of hydrogen from typical waste materials such as are found in municipal waste sites and sewage treatment plants, in amounts such that the chemically stored potential energy of the hydrogen produced exceeds the energy required to generate the electric current, whilst simultaneously reducing the mass of the waste material and/or reducing the time required to treat or dispose of such material. The invention thereby provides a method of hydrogen production which does not require the use of fossil fuels, which does not depend on the somewhat random appearance of sunlight, and which may, for example, be used to provide communities having relatively undeveloped electricity distribution and other energy infrastructures with a system that provides useful energy from collected wastes.

It is of particular interest to treat landfill materials because these materials present municipalities around the world with ubiquitous problems as breeding places for vectors such as rodents, cockroaches and communicable disease germs and as sources of greenhouse gases and groundwater contamination due to production of poisonous leachates. Treatment in accordance with the invention permits carbon sequestration from landfills, including those that are depositories for sewage sludge.

Thus according to one aspect of the invention there is provided a process for treating anaerobically decomposed organic material which comprises generating an electric current within said material so as to enhance hydrogen-producing microorganismal activity and to reduce methane-producing microorganismal activity, wherein an electric potential of 3-6 volts is employed.

Anaerobically decomposed organic materials which may be treated in accordance with this aspect of the invention include anaerobically composted cellulosic materials and anaerobically digested sewage sludge. Anaerobically composted cellulosic materials are typically found in landfill materials, which generally consist of approximately 70% cellulosic materials and have a moisture content of 36% to 46%. Anaerobically digested sewage sludge typically comprises sludge found at municipal sewage treatment plants; it is primarily liquid and includes 2-3% solids. Both landfill materials and sewage sludge naturally contain methane-producing bacterial species and hydrogen-producing bacterial species.

Other organic materials which may be treated include farm manure, crop waste and garbage. Such materials may advantageously be inoculated with, for example, samples of landfill material or sewage sludge prior to electric treatment. The inocula so used may if desired be pretreated by generation of an electric current therein or by digestion in the presence of an elevated concentration of hydrogen.

Anaerobic decomposition refers to a process where organic compounds, e.g. carbohydrates of the general formula CₙH₂ₙOₙ and other nutrients, decompose in the absence of an oxygen-donor environment. Volatile carboxylic acids such as acetic acid are typically formed in relatively high amounts by such anaerobic decomposition; ammonium bicarbonate is another typical degradation product. Although anaerobic decomposition may in some instances be preceded by aerobic decomposition, this is not a prerequisite for anaerobically decomposed organic material to be treated in accordance with this aspect of the invention. It will be appreciated that the process may be applied to organic materials in various stages of anaerobic decomposition and that generation of an electric current may be commenced prior to or at the onset of such decomposition.

Without wishing to be bound by theory, it is believed that the electric current promotes hydrolysis of the volatile carboxylic acids, which are known to act as electrolytes, and possibly also ammonium bicarbonate, thereby producing hydrogen. Since oxygen production is not observed, it would appear that electrolysis of water is not involved in such production of hydrogen. It is further believed that hydrogen so produced inhibits the subdivision, growth and activity of methanogenic species, whilst the production of hydrogen-releasing enzymes is greatly encouraged.

This aspect of the present invention may, for example, be practised at any large municipal landfill or sewage treatment facility, for example being continuously run in sewage digestion tanks. It may also be practised on a smaller scale wherever anaerobically decomposed organic materials such as anaerobically composted cellulosic materials or anaerobically digested sewage sludge are found or may be generated. Thus, for example, cellulosic materials and/or sewage sludge may be made to decompose "on-site", e.g. in a localized bin or chamber, rather than at a centralized landfill or sewage treatment facility. Such anaerobically decomposed organic materials may then optionally be taken to a transfer station equipped to produce hydrogen by treatment in accordance with the invention, or alternatively may be made to produce hydrogen "on-site" by treatment at or near the on-site bin or chamber. In such embodiments, hydrogen may be stored or may be used on-site to produce useful forms of energy, including the relatively minor amount of energy required to generate the electric current.

The electric current may, for example, be generated by application of an electric potential across electrodes in contact with the organic material, for example one or more sets of electrodes placed within the material. Such electrodes may, for example, be made from lead, copper, steel, brass, or carbon, more preferably from cast iron bars, and most preferably from metal-impregnated or other forms of graphite having enhanced electrical conductivity. Electrode sets may be of any suitable shape, e.g. plates, bars, grids, etc.

Treatment in accordance with this embodiment of the invention is conveniently effected by application of an electric potential of between 3 and 6 volts, preferably between 3.0 and 4.5 volts.

It is desirable to generate an electric current with low polarization and ohmic losses. Electrode placement and separation may be adjusted to achieve these conditions, and it may also be advantageous to adopt a program of voltage control including occasional reversal of polarity. The voltage, average spacing of electrodes and number of electrodes may be varied depending upon the size and composition of the organic material to be treated.

In a preferred embodiment of this aspect of the invention, each individual electrode is placed into landfill material and is surrounded by an inert "cage" which effectively ensures that the moisture component of the landfill material, and not a component which might interfere with electrical activity, is immediately adjacent each electrode. It will be appreciated that optimum placement of each electrode within the landfill material may require some trial and error.

The electric current may advantageously be generated intermittently, preferably at intervals selected to minimize electric power consumption whilst maximizing hydrogen production, e.g. at intervals determined with reference to levels of hydrogen and/or methane detected in gas produced from the organic material. Thus, for example, a duty cycle of electric potential application may be adaptively adjusted by reference to feedback information from a gas detector and associated controller means. In one such embodiment an electric potential is applied when trace amounts of methane are detected and is maintained until methane production is substantially completely suppressed; the time period involved is recorded. Following cessation of the application of electric potential the controller then records the time period which occurs until methane traces are again detected and thereafter sets a duty cycle in which electric potential is applied for a time interval slightly longer than the time recorded for suppressing methane production and is switched off for a time interval slightly less than the time recorded for redetection of traces of methane. The voltage of the applied electric potential may if desired be controllably reduced as part of the adaptively adjusted control in order to minimize energy expenditure.

In a typical example of a treatment process in accordance with this aspect of the invention, hydrogen production begins upon generation of an electric current within the anaerobically decomposed organic material and increases to from 70% to 75% by volume of the total gases produced. The level of methane produced decreases from a high of approximately 70% by volume of the total gases produced, when the electric current is first applied, to greatly diminished trace levels. Carbon dioxide and nitrogen production remain relatively constant and do not vary significantly with methane or hydrogen production.

Substantial amounts of carbon dioxide are produced along with hydrogen from anaerobically decomposed organic material treated in accordance with the invention. Economic separation of the hydrogen from this carbon dioxide is desirable in order to increase the value of the hydrogen, for example by increasing its storage density and facilitating its use in fuel cell applications. Separated carbon dioxide may, for example, be utilized in greenhouses or hydroponics.

Carbon dioxide produced from the organic material is separated from hydrogen produced from the organic material by preferential absorption of said carbon dioxide in a pressurized fluid whereby said hydrogen escapes to collection means.

The pressurized fluid is advantageously water. The solubility of carbon dioxide in water is about 21.6 volumes of gas per volume of water at 25 atmospheres pressure and 12°C (54°F). Increasing the pressure or decreasing the temperature increases the amount of carbon dioxide dissolved per volume of water, whilst lowering the pressure or increasing the temperature releases dissolved carbon dioxide. In most areas of the Earth, ground water is maintained at a temperature that is equal to the mean annual air temperature plus 0.55°C (1.0°F) for each 24.4 metres (80') of overburden of soil strata to the saturated zone.

The mixed gases, e.g. consisting of hydrogen, carbon dioxide and lesser amounts of nitrogen and other gases, may for example be forced into the bottom of a column of water having a height of at least 300 metres (approximately 1,000'), maintained at a temperature in the range 4-16°C (40-60°F). Such a column may, for example, comprise a well extending 300 metres below the saturated zone of the local groundwater. This provides the extremely large heat sink benefit of the sub soil, including the ground water in the saturated zone, where the temperature is generally constant within the desired temperature range for most climate zones throughout the year. Water columns that are elevated along mountain slopes are also feasible but may suffer freezing conditions in the winter and unfavorable warming in the summer season.

Carbon dioxide readily enters into solution at the pressure and temperature conditions maintained, whilst gaseous hydrogen may be collected and delivered to the surface for various uses.

As described in greater detail hereinafter, carbon dioxide-containing pressurized fluid may be withdrawn and, optionally after addition of heat, permitted to expand to allow release of carbon dioxide and to transfer energy to a motor. The expanded fluid may subsequently be cooled, pressurized and recycled for further absorption of carbon dioxide.

### Brief Description of the Drawings

In the accompanying drawings, which illustrate the invention without in any way limiting the same:

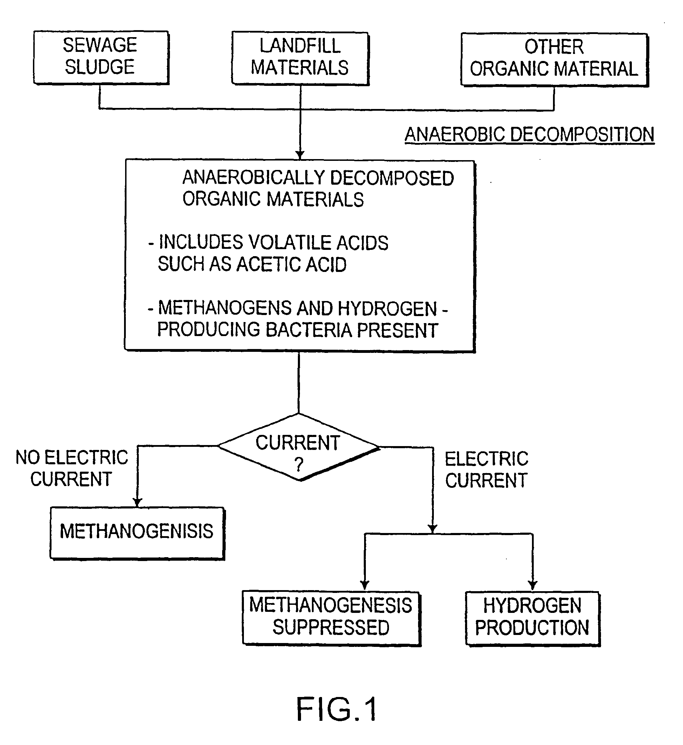

Fig. 1 is a flow chart showing both production of hydrogen and suppression of methanogenesis from anaerobically decomposed organic materials in the presence of an applied electropotential, and methanogenesis from anaerobically decomposed organic materials in the absence of an applied electropotential;

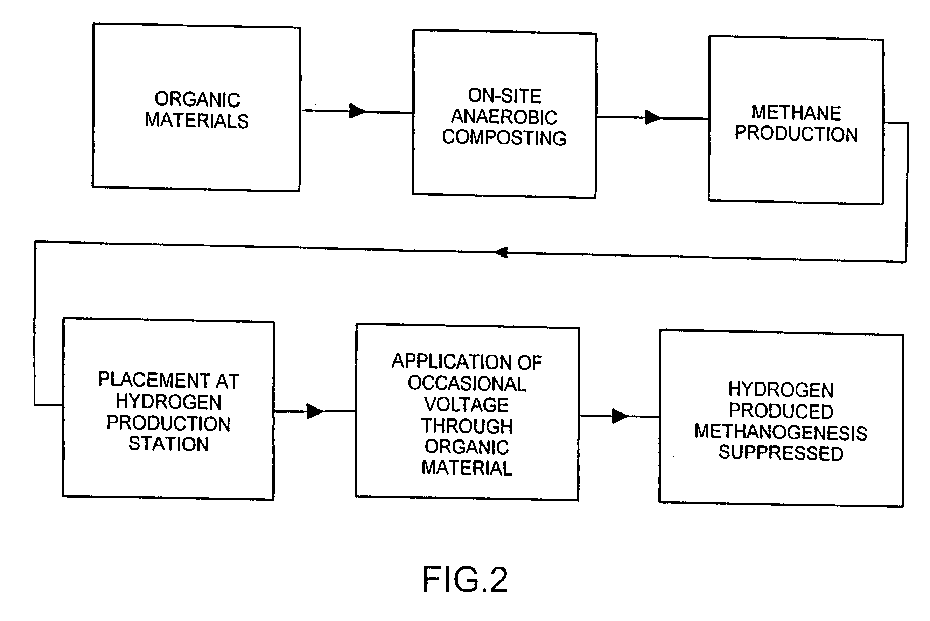

Fig. 2 is a flow chart showing a process for production of hydrogen which includes on-site anaerobic decomposition of organic material;
Fig. 3 is a bar graph representation of the information in Table 1 of Example 1;
Fig. 4 is a bar graph representation of the information in Table 2 of Example 2;
Fig. 5 is a bar graph representation of the information in Table 3 of Example 3;
Fig. 6 is a bar graph representation of the information in Table 5 of Example 5;
Fig. 7 is a bar graph representation of the information in Table 6 of Example 5;
Fig. 8 is a bar graph representation of the information in Table 8 of Example 6;
Fig. 9 is a bar graph representation of the information in Table 9 of Example 7;
Fig. 10 is a bar graph representation of the information in Table 10 of Example 8;
Fig. 11 is a schematic illustration of a system that adaptively controls application of intermittently applied voltage to maximize hydrogen production while minimizing methane production;
Fig. 12 is a schematically illustrated system showing generation of voltage for production of hydrogen in accordance with the invention;
Fig. 13 is a schematic illustration of another system; and
Fig. 14 is a schematic illustration of a still further system.

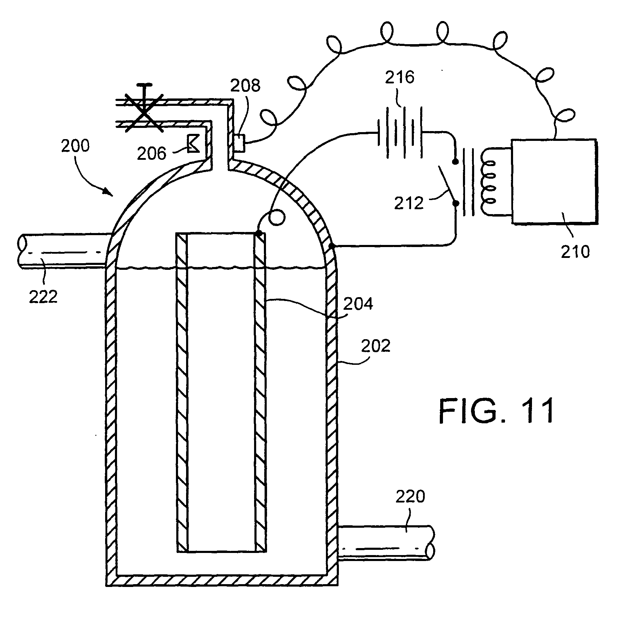

Referring to the drawings in more detail, Fig. 11 shows an embodiment 200 in which suitable electrodes such as concentric electrodes 202 and 204 receive intermittently applied voltage to influence solvated organic waste between the electrodes to produce hydrogen. In operation, voltage is applied by voltage source 216 according to a duty cycle controlled by relay 212, which is constantly adjusted by controller 210 to facilitate hydrogen generation and to prevent substantial methane production.

Feedback information from gas detector 206/208 is provided to controller 210. If trace amounts of methane are detected, a voltage is applied between electrodes 202 and 204 for a recorded time period until methane production is depressed. The time until methane traces are detected again is noted by controller 210 and a duty cycle of applying voltage across electrodes 202 and 204 for a time interval slightly longer than the time noted for depressing methane production, followed by neutral electrode operation for a time period slightly less than the time noted previously for traces of methane to be detected, is adopted.

This duty cycle is adaptively changed to shorten the time of voltage application and to extend the time between voltage application for purposes of minimizing methane production while maximizing hydrogen production with least application of voltage to electrodes 202 and 204. Voltage level is reduced to provide another variable and adaptively adjusted with respect to the time of voltage application to minimize energy expenditure. This adaptive control algorithm rapidly adjusts for changes in organic waste composition, moisture content, temperature and other variables.

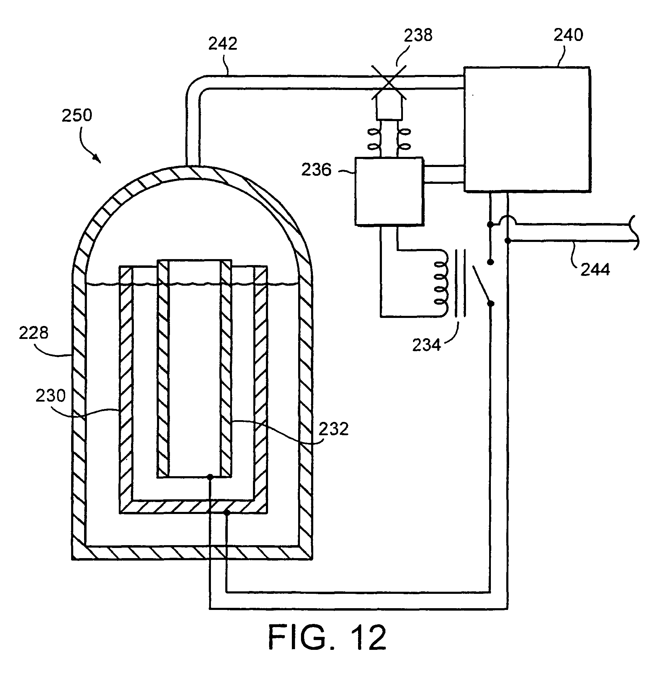

Fig. 12 shows an embodiment in which the fuel gas produced by the process of the invention in the presence of electrodes 230 and 232 is in part made available for conversion to electricity by a fuel cell or engine-generator set 240. Adaptively controlled application of voltage to electrodes 230 and 232 is provided by controller 236 and relay 234 as shown for purposes of minimizing energy consumption per therm of hydrogen produced. Moreover, adaptive controller 236 provides a control algorithm to minimize methane production while facilitating maximum hydrogen production. Solenoid operated valve 238 controls delivery of fuel gas by line 242 to energy conversion unit 240 as needed to meet an adaptively adjusted duty cycle and to meet other demands for electricity as delivered by insulated cables 244. Suitable power for pumping water, providing a heat-pump cycle, or production of electricity at 240 may be by a heat engine and generator, a fuel cell, a thermoelectric generator, or other devices that convert fuel potential energy into electricity.

In many applications, it is preferred to utilize a piston engine and generator in which the engine is fuelled with a SmartPlug combination fuel injector and ignition system, since this facilitates extremely robust operation. SmartPlug operation is disclosed in U.S. Patents Nos. 5,394,852 and 5,343,699, and enables a raw mixture of hydrogen and carbon dioxide to be used as a very low grade fuel without further conditioning, while producing very high thermal efficiency and full rated power in comparison with engine operation on gasoline or diesel fuel. This is a particularly important advantage for remote operation and for bringing fuel and power to depressed economies where it is prohibitive to import fossil-based fuels.

Preferential production of hydrogen provides thermodynamic advantages based on faster fuel combustion, wider air/fuel ratio combustion limits, and with SmartPlug operation the engine operates essentially without throttle losses. These thermodynamic advantages provide much higher brake mean effective pressure (BMEP) for the same heat release in comparisons with gasoline or diesel fuel.

As shown in Table A, it is possible actually to clean ambient air with an engine generator running on hydrogen-characterized fuel produced from landfill or sewage organic wastes, in contrast to operation using gasoline as fuel.

**Table A**

| **Gas** | **HC (ppm)** | **CO (ppm)** | **NO (ppm)** |
|---|---|---|---|
| Ambient air | 29 | 0.00 | 1.0 |
| Exhaust from hydrogen-fuelled engine, idling | 18 | 0.00 | 1.0 |
| Exhaust from hydrogen-fuelled engine, full power | 6 | 0.00 | 2.0 |
| Exhaust from gasoline-fuelled engine, idling | 190 | 25,000 | 390 |
| Exhaust from gasoline-fuelled engine, full power | 196 | 7,000 | 95 |

| | | | |
|---|---|---|---|
| Key - HC = hydrocarbons; CO = carbon monoxide; NO = nitric oxide. | | | |

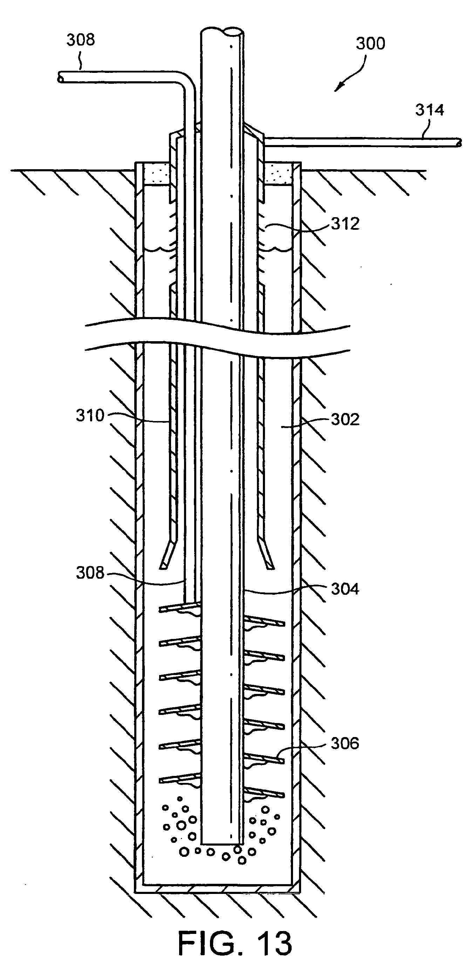

Fig. 13 shows a system for separating carbon dioxide from hydrogen by differential absorption of carbon dioxide within a suitable medium such as water or a hindered amine. In operation, mixed gases consisting of hydrogen, carbon dioxide and lesser amounts of nitrogen and other gases are forced into the bottom of a column of water 302 approximately 300 metres (1,000') or more in height.

Mixed gases are delivered to the bottom of tube 304 by a suitable pump (not shown), and enter into a suitable scrubber zone such as the helical fin 306, which is attached to tube 304 with a higher elevation at the point of attachment than any other point on the element of rotation that describes the helical surface as shown. Gases thus tend to be buoyed towards tube 304 as they are scrubbed by the absorbing fluid. Carbon dioxide readily enters into solution at the pressure and temperature conditions maintained. Hydrogen exits at the top of the helix into tube 308 and is delivered to the surface for various uses.

Carbon dioxide-rich water is ducted to the surface by coaxial tube 310 as shown. As the head pressure lessens, carbon dioxide bubbles develop and escape upward and create a lower density mixture that is buoyantly lifted to the gas separator section 312, where denser water that has lost the ability to retain carbon dioxide is returned to annular space 302 and sinks the bottom to replace the upward travelling inventory of water that is lifted within tube 310. Carbon dioxide is collected at the top of 310 by tube 314.

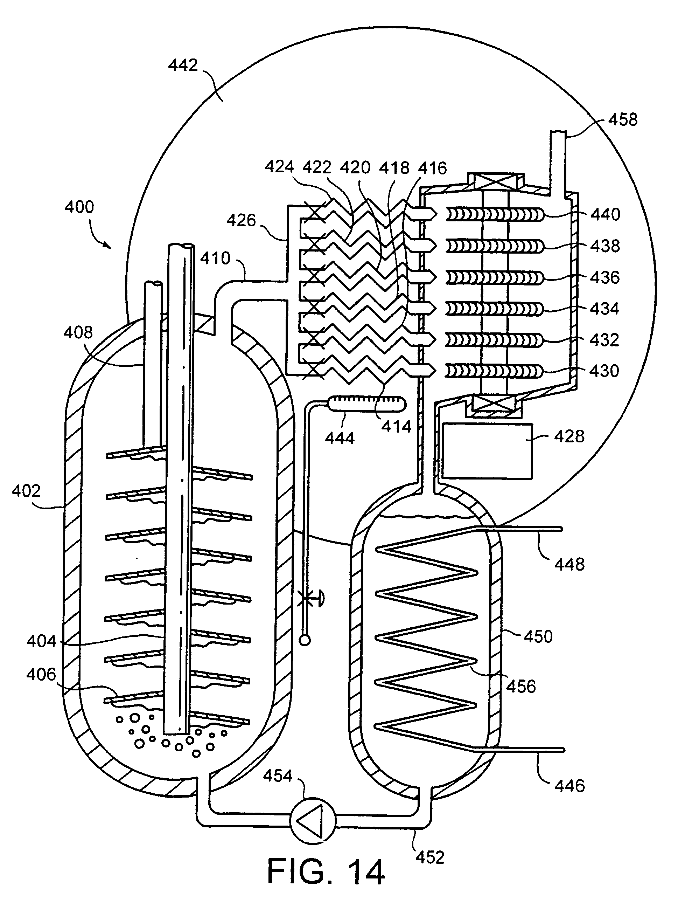

Fig. 14 shows an embodiment in which energy used to pressurize the hydrogen and carbon dioxide is regeneratively recovered by an expansion engine. Embodiment 400 shows an extremely rugged and simple energy conversion system that combines various renewable resources such as sewage, garbage and farm wastes with solar energy to supply electricity, hydrogen and carbon dioxide.

In many situations and applications it is preferred to pressurize water in a suitable vessel 402 to provide for the separation by solubility differences as desired to purify hydrogen. In operation, mixtures of hydrogen and carbon dioxide are forced through tube 404 into pressure vessel 402 at the nominal pressure of 3100 kPa (450 psi). It is preferred to utilize a spiral mixer consisting of a helical fin 406 that causes the mixture of gases to scrub along the surface and form high surface-to-volume ratios. The mixed gases follow an extended path through the water as carbon dioxide is absorbed to allow the hydrogen to be collected at the top of spiral scrubber 406 by tube 408 as shown. Carbon dioxide is absorbed into the water while hydrogen is collected at the top of separator 406 as shown.

Hydrogen is delivered by conduit 408 for immediate use in an engine or fuel cell or it may be stored for future use as needed. Carbon dioxide-saturated water is taken from absorber vessel 402 by tube 410 to valve manifold 426, which provides control valves to time the flow of carbon dioxide-rich water into each of a group of heat exchangers such as 414, 416, 418, 420, 422 and 424 as shown. Each heat exchanger is provided with an exit nozzle that is aimed at the blades or buckets of an adjacent fluid motor rotor such as 430, 432, 434, 436, 438 and 440, which deliver work to a common output shaft as shown.

An inventory of water and carbon dioxide solution under pressure is suddenly forced into a preheated heat exchanger such as 414 by briefly opening the control valve that serves 414. As the fluid is heated, the temperature and pressure of the fluid increases and it vaporizes and is expelled with very high momentum to power motor 430. Each of the other heat exchanger chambers receives a charge of fluid on a timed basis so that the shaft power from the group of motors shown can be considered to have multiple phase torquing, such as six phase if each heat exchanger receives flow at a different times, or three phase if two heat exchangers are filled simultaneously. A suitable application of the output of the fluid motor is generator 428 or other useful loads as needed.

It is preferred to provide concentrated radiation to the heat exchangers by a suitable solar collector such as a field of heliostats or a parabolic dish 442 as shown. At times when solar energy is insufficient to meet energy conversion needs, supplemental heat may be applied by combustion from a suitable burner 448. For such supplemental heating it is preferred to use mixtures of carbon dioxide and hydrogen and/or other combustible gases released by anaerobic digestion of organic matter.

After undergoing heating and expansion to a suitably low pressure, carbon dioxide is collected by tube 458 and taken to a suitable application. Water is condensed and collected in reservoir 450 which is cooled by countercurrent heat exchanger 456 by circulation of a suitable heat exchange fluid from 446 to 456 and then through 448 to a suitable cogeneration application. Cooled water is pressurized by pump 454 and returned to pressure vessel 402 to complete the novel carbon dioxide removal and energy conversion cycle.

The following non-limitative Examples serve to illustrate the invention.

### Materials and methods

Electrodes were cast iron bars, 300 mm long, 25 mm wide and 2.5 mm thick. Other metallic electrodes were also used, including lead, copper, steel and brass electrodes. A pair of copper impregnated graphite electrodes of the same size was also used; degradation of the graphite electrode was not very noticeable.

Samples of landfill material were obtained from a sanitary landfill at Staten Island, New York from a depth of 9.1 to 15.2 metres (30 to 50 feet). The landfill materials naturally produced methane and carbon dioxide as primary gases (in 55:35 proportions) through methanogenesis, and had a pH of 6.5-7.0.

Sludge samples were taken from a primary digester of a sewage treatment plant at Brooklyn, New York. The sewage sludge naturally produced methane and carbon dioxide (in 65:30 proportions) by methanogenesis, and had a pH of 7.0-7.5.

In one set of experiments each sample was investigated in an 800 ml flask with a three hole rubber stopper. Electrodes were inserted into the sample through two of the holes; the third hole was fitted with a glass delivery tube connected to a gas analyzer. The electrodes were connected across two 1.5 volt batteries in series, resulting in an applied potential of about 3.0 volts. The apparatus was placed in an incubator set either at 37°C or at 55°C. Other experiments were performed using a New Brunswick Fermenter having a 6-8 liter glass vessel where the temperature and rate of stirring could be controlled as desired.

### Example 1

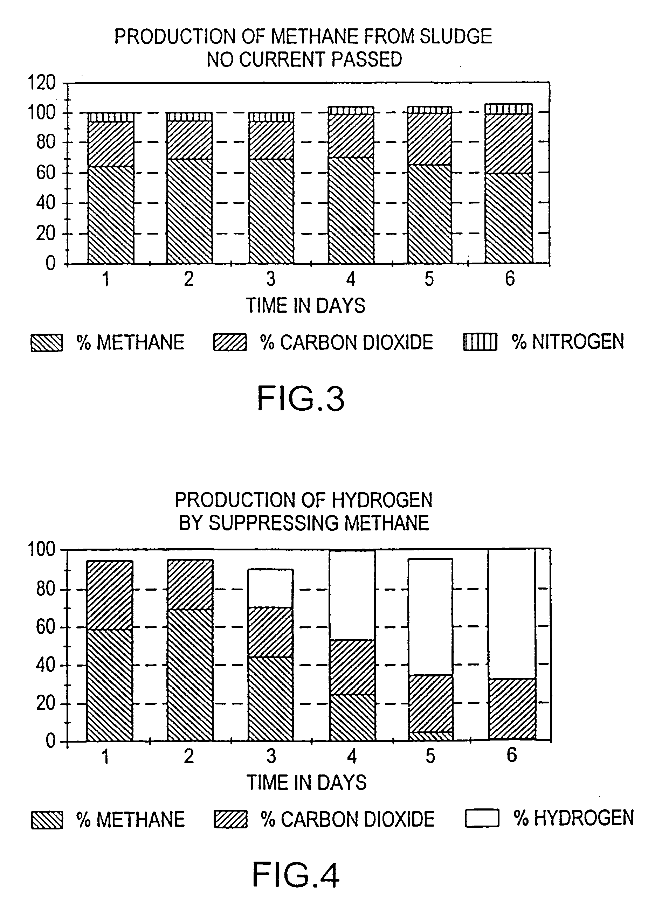

As an experimental control, freshly obtained sewage sludge in an 800 ml flask was placed in an incubator at 37°C. Gases, primarily methane, were produced as described in Table 1 and depicted in Fig. 3.

**Table 1 - Production of methane and carbon dioxide**

| nays | %CH₄ | %CO₂ | %N₂ |
|---|---|---|---|
| 1 | 65 | 30 | 5 |
| 2 | 70 | 25 | 5 |
| 3 | 70 | 25 | 5 |
| 4 | 65 | 30 | 4 |
| 5 | 60 | 35 | 4 |
| 6 | 55 | 40 | 5 |

### Example 2

Sewage sludge from the primary digester was placed in an 800 ml flask which was then placed in a preheated incubator at 37°C. Methane gas was generated. As soon as optimum production of methane was achieved, an electric current was passed through the liquid in the flask. The production of methane gas declined gradually and hydrogen and carbon dioxide were produced. Methane was completely suppressed when production of hydrogen reached its peak, as described in Table 2 and depicted in Fig. 4.

**Table 2 - Production of hydrogen and suppression of methane**

| Days | %CH₄ | I %CO₂ | %H₂ |
|---|---|---|---|
| 1 | 60 | 35 | - |
| 2 | 70 | 25 | - |
| 3* | 45 | 25 | 20 |
| 4 | 25 | 28 | 46 |
| 5 | 5 | 30 | 60 |
| 6 | trace | 30 | 68 |

| | | | |
|---|---|---|---|
| * onset of passage of electric current | | | |

### Example 3

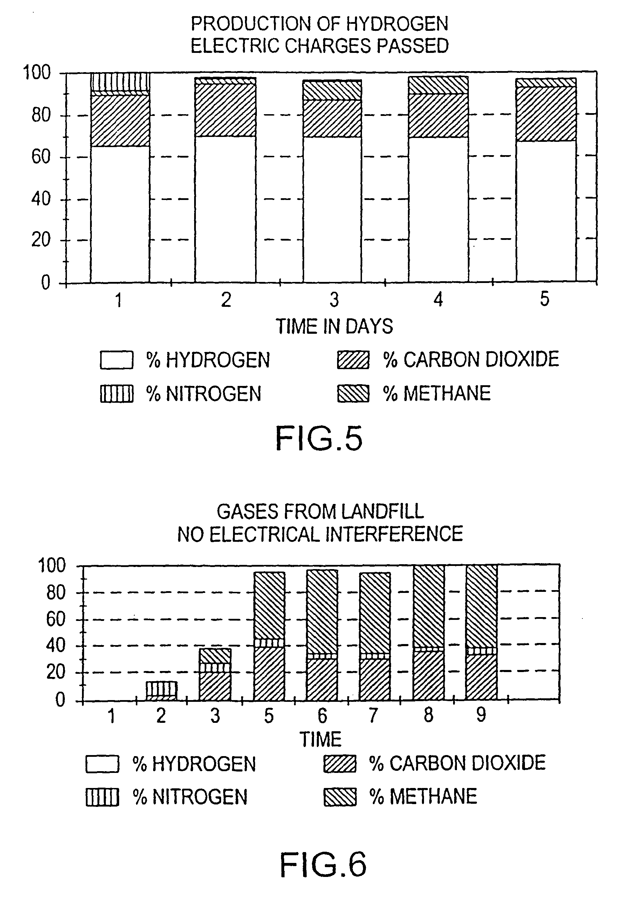

Sewage sludge from the primary digester was placed in an 800 ml flask which was then placed in an incubator at 37°C. An electric current was passed through the sludge, applying 3 volts using the two 1.5 volt batteries in series. Very little methane was produced from the outset. Within 3 days, production of hydrogen reached its peak and methane gas was virtually totally suppressed, as described in Table 3 and depicted in Fig. 5.

**Table 3 - Production of hydrogen and carbon dioxide when voltage applied from start**

| Days | %H₂ | %CO₂ | %N₂ | %CH₄ |
|---|---|---|---|---|
| 1 | 65 | 25 | 2 | 8 |
| 2 | 70 | 25 | 2 | trace |
| 3 | 70 | 18 | 8 | trace |
| 4 | 70 | 20 | 8 | - |
| 5 | 68 | 25 | 4 | - |

### Example 4

A sewage sludge sample was placed in a 5 liter flask in the New Brunswick Fermenter and 4 electrodes were introduced. An electric current (2.5 volts and .05 to .07 amps) was passed through the sample. Initially only methane and carbon dioxide were produced, with very little hydrogen being generated. As soon as the voltage was increased to 4.0 - 4.5, and current to 0.11 - 0.15 amps, methane was gradually suppressed and hydrogen was produced as described in Table 4.

**Table 4 - Production of hydrogen and carbon dioxide from sewage sludge in 5 liter container**

| Days | %H₂ | %CO₂ | %N₂ | %CH₄ |
|---|---|---|---|---|
| 1 | - | 30 | 12 | 50 |
| 2 | 5 | 35 | 8 | 46 |
| 3 | 4 | 30 | 6 | 60 |
| 5 | 25 | 30 | 5 | 40 |
| 6 | 48 | 25 | 5 | 20 |
| 7 | 60 | 20 | 2 | 8 |
| 9 | 70 | 25 | 4 | trace |

### Example 5

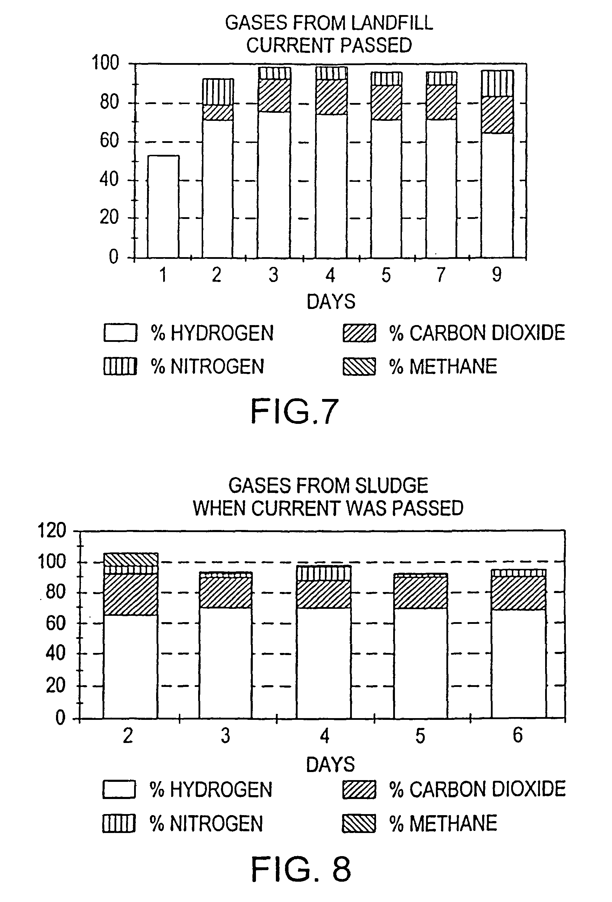

Landfill materials collected by random borings were examined for determination of the least energy expenditures per unit of energy produced. Experiments were set up with landfill materials (composted municipal solid wastes) in two 800 ml flasks, (1) with landfill materials only, (2) with electrically treated landfill materials. The results are described in Tables 5 and 6 and depicted in Figs. 6 and 7.

**Table 5 - Production of gases from landfill materials**

| Days | %H₂ | %CO₂ | %N₂ | %CH₄ |
|---|---|---|---|---|
| 1 | - | - | - | - |
| 2 | - | 3 | 10 | - |
| 3 | - | 20 | 8 | 10 |
| 5 | - | 40 | 6 | 50 |
| 6 | - | 30 | 5 | 63 |
| 7 | - | 30 | 5 | 60 |
| 8 | - | 35 | 4 | 60 |
| 9 | - | 35 | 5 | 62 |

**Table 6 - Production of gases from electrically treated landfill materials**

| Days | %H₂ | %CO₂ | %N₂ | %CH₂ | Total gas volume (cc) |
|---|---|---|---|---|---|
| 1 | 53 | - | - | - | 95 |
| 2 | 72 | 8 | 13 | - | 302 |
| 3 | 76 | 17 | 6 | - | 500 |
| 4 | 75 | 18 | 6 | - | 600 |
| 5 | 72 | 18 | 6 | - | 450 |
| 7 | 72 | 18 | 6 | - | 600 |
| 9 | 65 | 18 | 14 | - | 500 |

### Example 6

The procedure of Example 5 was repeated (1) with sewage sludge only, (2) with electrically treated sewage sludge. The results are described in Tables 7 and 8 and depicted in Fig. 8.

**Table 7 - Production of gases from sewage sludge**

| Days | %H₂ | %CO₂ | %N₂ | %CH₄ | Total gas volume (cc) |
|---|---|---|---|---|---|
| 2 | - | 20 | 14 | 65 | 50 |
| 3 | - | 14 | 10 | 70 | 125 |
| 4 | - | 19 | 4 | 72 | 225 |
| 5 | - | 22 | 4 | 66 | 258 |
| 6 | - | 18 | 8 | 70 | 200 |

**Table 8 - Production of gases from electrically treated sewage sludge**

| Days | I %H₂ | I %CO₂ | %N₂ | %CH₄ | Total gas volume (cc) |
|---|---|---|---|---|---|
| 2 | 65 | 28 | 4 | 8 | 85 |
| 3 | 70 | 20 | 2 | trace | 200 |
| 4 | 70 | 18 | 8 | trace | 310 |
| 5 | 70 | 20 | 2 | - | 330 |
| 6 | 68 | 22 | 4 | - | 258 |

### Example 7

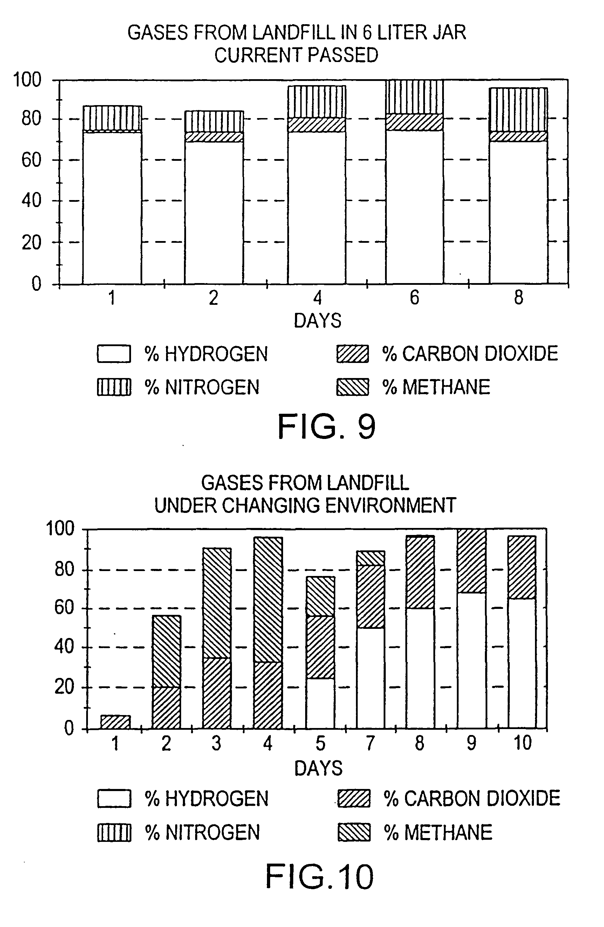

A current was passed through landfill materials in a 6 liter vessel equipped with electrodes by applying an electric potential of 3.5 volts. The results are described in Table 9 and depicted in Fig. 9.

**Table 9 - Production of gases from landfill materials in 6 liter vessel**

| Days | %H₂ | %CO₂ | %N₂ | %CH₄ | Total gas volume (cc) |
|---|---|---|---|---|---|
| 1 | 75 | trace. | 12 | - | 100 |
| 2 | 70 | 5 | 10 | - | 1020 |
| 4 | 75 | 7 | 15 | - | 850 |
| 6 | 75 | 8 | 17 | - | 750 |
| 8 | 70 | 5 | 20 | - | 600 |

### Example 8

Landfill materials in a 6 liter vessel equipped with electrodes were placed in a preheated incubator at 55°C. After 4 days an electric potential of 3.5 volts was applied across the electrodes. The results are described in Table 10 and depicted in Fig. 10.

**Table 10 - Production of gas from landfill materials**

| Days | %H₂ | %CO₂ | %N₂ | %CH₄ | Total gas volume (cc) |
|---|---|---|---|---|---|
| 1 | - | 5 | - | - | 20 |
| 2 | - | 20 | - | 35 | 125 |
| 3 | - | 35 | - | 55 | 200 |
| 4 | | | | | |
| 5* | - | 30 | - | 20 | 150 |
| 7 | 25 | 31 | - | 7 | 150 |
| 8 | 60 | 35 | - | trace | 250 |
| 9 | 68 | 31 | - | - | 285 |
| 10 | 65 | 30 | - | - | 200 |

| | | | | | |
|---|---|---|---|---|---|
| * onset of passage of electric current | | | | | |

Similar results were achieved by mixing relatively small amounts of inocula of human sewage sludge with farm manure and/or crop wastes. After incubation periods in which anaerobic conditions were established, methane and carbon dioxide were produced with very little hydrogen. Upon application of an electric potential of 2.0 to 5.0 volts to generate a current of 0.10 to 0.20 amps, methane production was depressed and hydrogen was produced in similar amounts to those shown in Table 10. Similar results were also achieved using inocula from previous runs of Example 4.

## Claims

1. A process for treating anaerobically decomposed organic material which comprises generating an electric current within said material so as to enhance hydrogen-producing microorganismal activity and to reduce methane-producing microorganismal activity, wherein an electric potential of 3-6 volts is employed.

2. A process as claimed in claim 1 wherein said anaerobically decomposed organic material comprises anaerobically composted cellulosic materials and/or anaerobically digested sewage sludge.

3. A process as claimed in claim 1 or claim 2 wherein said electric current is generated by application of an electric potential across electrodes in contact with the organic material.

4. A process as claimed in any of the preceding claims wherein a portion of hydrogen produced from the organic material is used in an energy conversion process to provide energy for generating said electric current.

5. A process as claimed in any of the preceding claims wherein said electric current is generated intermittently.

## Patentansprüche

1. Verfahren zur Behandlung von anaerob zersetztem organischem Material, das das Erzeugen eines elektrischen Stroms im Material umfasst, um die Aktivität wasserstofferzeugender Mikroorganismen zu steigern und die Aktivität methanerzeugender Mikroorganismen zu reduzieren, worin ein elektrisches Potential von 3-6 Volt eingesetzt wird.

2. Verfahren gemäß Anspruch 1, worin das anaerob zersetzte organische Material anaerob kompostierte Zellulosematerialien und/oder anaerob abgebauten Klärschlamm umfasst.

3. Verfahren gemäß Anspruch 1 oder 2, worin der elektrische Strom durch Anlegen eines elektrischen Potentials über Elektroden im Kontakt mit dem organischen Material erzeugt wird.

4. Verfahren gemäß einem der vorhergehenden Ansprüche, worin ein Teil des aus dem organischen Material erzeugten Wasserstoffs in einem Energierumwandlungsprozess verwendet wird, um Energie zur Erzeugung des elektrischen Stroms bereitzustellen.

5. Verfahren gemäß einem der vorhergehenden Ansprüche, worin der elektrische Strom unterbrochen erzeugt wird.

## Revendications

1. Procédé pour traiter une matière organique décomposée de manière anaérobie qui comprend de générer un courant électrique à l'intérieur de ladite matière de manière à augmenter l'activité des microorganismes pour la production d'hydrogène et pour diminuer l'activité des microorganismes pour la production de méthane, dans lequel un potentiel électrique de 3 - 6 volts est utilisé.

2. Procédé tel que revendiqué dans la revendication 1 dans lequel ladite matière organique décomposée de manière anaérobie comprend des matières cellulosiques compostées de manière anaérobie et/ou des boues d'épuration digérées de manière anaérobie.

3. Procédé tel que revendiqué dans la revendication 1 ou la revendication 2 dans lequel ledit courant électrique est généré par application d'un potentiel électrique à travers des électrodes en contact avec la matière organique.

4. Procédé tel que revendiqué dans l'une quelconque des revendications précédentes dans lequel une portion d'hydrogène produit à partir de la matière organique est utilisée dans un procédé de conversion d'énergie pour fournir de l'énergie pour générer ledit courant électrique.

5. Procédé tel que revendiqué dans l'une quelconque des revendications précédentes dans lequel ledit courant électrique est généré de manière intermittente.
